(19) Europäisches Patentamt
European Patent Office
Office européen des brevets

(11) Publication number : **0 259 371 B1**

(12) # EUROPEAN PATENT SPECIFICATION

(45) Date of publication of patent specification : **13.05.92 Bulletin 92/20**

(21) Application number : **87901068.4**

(22) Date of filing : **28.01.87**

(86) International application number : **PCT/GB87/00057**

(87) International publication number : **WO 87/04433 30.07.87 Gazette 87/17**

(51) Int. Cl.$^5$ : **C07D 253/08,** C07D 487/04, C07C 277/02, // (C07D487/04, 253:00, 331:00)

(54) **PREPARATION OF 3-DIMETHYLAMINO-7-METHYL-1,2,4-BENZOTRIAZINE-1-OXIDE AND AZAPROPAZONE.**

(30) Priority : **28.01.86 GB 8602035**
**28.01.86 GB 8602037**
**15.01.87 GB 8700862**

(43) Date of publication of application : **16.03.88 Bulletin 88/11**

(45) Publication of the grant of the patent : **13.05.92 Bulletin 92/20**

(84) Designated Contracting States : **AT BE CH DE FR IT LI LU NL SE**

(56) References cited :
**FR-A- 1 440 629**
**Journal of Chemical Society,serie B,1970 (London,GB)J.C Mason et al., "Heterocyclic N-Oxides.Part VI.Synthesis and NuclearMagnetic Resonance Spectra of 3-Aminobenzo-1,2,4,-triazines and their Mono-and Di-Oxides", pages 911-916, see page 915,experimental part and formulas.**
**Journal of the American Society, vol.76,no 13, 5 July 1954 (Washington DC,USA), F.J Wolf et al., "Benzotriazines. I. A NewSeries of compounds Having Antimalarial Acticity", pages 3551-3553**

(56) References cited :
**Helvetica Chimica Acta, vol 55, fasc.3 , No 105, 1972 (Basle,CH), G. MIxich, "105. Isolierung, Struktur und Synthese desMetaboliten von Azapropazon-dihydrat", pages 1031-1038, see page 1034.**
**Chemical Abstracts,vol 105, no 14, 6 October 1986 (Columbus, Ohio, USA), P: Pazdera et al., "Electrochemical behaviour of2-nitrophenylguanidines in acetonitrile", see page 510, abstract no.122778s, J.Electroanal.Chem. Interfacial Electrochem.1986,207(1-2), 189-202**

(73) Proprietor : **JOHN WYETH & BROTHER LIMITED**
**Huntercombe Lane South Taplow**
**Maidenhead Berkshire, SL6 0PH (GB)**
Proprietor : **Siegfried Pharma A.G.**
**CH-4800 Zofingen (CH)**

(72) Inventor : **WALKER, Francis, S.**
**35 Friday Street Warnham**
**Horsham West Sussex RH12 3QY (GB)**
Inventor : **BENN, Frederick, Roger**
**159 Framingham Road Sale**
**Cheshire M3 3RQ (GB)**

(74) Representative : **Kearney, Kevin David Nicholas**
**KILBURN & STRODE 30 John Street**
**London, WC1N 2DD (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

This invention relates to an improved process for the preparation of 3-dimethylamino-7-methyl-1,2,4-benzotriazine-1-oxide, hereinafter referred to for simplicity as "benzotriazine oxide", and to benzotriazine oxide prepared by the improved process, and to azapropazone prepared therefrom.

Benzotriazine oxide is of commercial importance because it is an intermediate in the preparation of the anti-inflammatory drug azapropazone. The conventional preparation of benzotriazine oxide involves the reaction of 4-methyl-2-nitroaniline with phosgene and the subsequent treatment of the resulting urea derivative with ammonia to neutralise excess phosgene, followed by purification and treatment with sodium hydroxide. This procedure is disadvantageous in view of the highly toxic nature of phosgene and also because it is necessary to carry out the reaction in several stages involving separate reaction vessels.

A process has been known since 1913 for the preparation of benzotriazine oxide by reaction of a substituted nitrobenzene with cyanamide in the melt followed by addition of concentrated hydrochloric acid and then cyclization of the intermediate by reaction with alkali. (F. ARNDT Ber.dtsch Chem.Ges. 46 3522-3530 (1913)). This reaction was reported by Arndt as being extremely vigorous. In 1954 Wolf et al JACS 76, p.3551-3553 reviewed and repeated Arndt's work using cyanamide. They described the reaction as "violent". They modified the reaction to carry it out in glacial acetic acid but did not report yields greater than 50%.

In 1970 Mason and Tennant J. Chem. Soc. Series B. 1970 p. 911-916 used Arndt's reaction to produce 3-amino-7-methyl-1,2,4-benzotriazine-1-oxide in 66% yield. However, to produce 3-dimethylamino-7-methyl-1,2,4-benzotriazine-1-oxide this compound would have to be dimethylated which would result in a significantly lower yield of the required dimethylamino compound.

We have found that the use of dimethylcyanamide in solution in a non-polar solvent with hydrogen chloride gas bubbled through results in a smooth safe reaction with a good yield.

The preparation of the guanidinyl compound preferably involves the reaction of a compound having the formula:

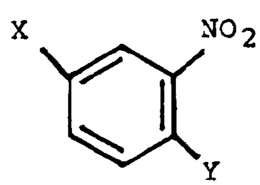

with a cyanamide, wherein Y represents a group reactive with the said cyanamide to produce a compound having the formula:

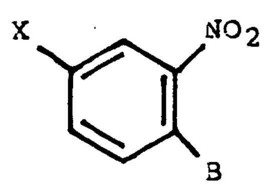

wherein B represents an NCN grouping.

The NCN grouping may be of the formula:

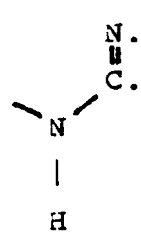

According to the present invention, a process for the preparation of 3-dimethylamino-7-methyl-1,2,4-benzotriazine-1-oxide comprises providing a solution in a non-polar solvent which at 20°C contains 1 to 30% by weight of dimethylcyanamide and 5 to 50% by weight of 4-methyl-2-nitroaniline and heating the solution with the introduction of gaseous hydrogen chloride at a temperature above 100°C and less than 160°C to cause the cyanamide and the aniline compound to react to produce a precipitate of N,N-dimethyl-N'-(4-methyl-2-nitrophenyl) guanidine hydrochloride, which is then dehydroxylated by being heated with an alkali to produce 3-dimethylamino-7-methyl-1,2,4-benzotriazine-1-oxide.

The non-polar solvent for the 4-methyl-2-nitroaniline and the dimethylcyanamide is preferably toluene. The initial reaction mixture at 20°C may conveniently contain 15 to 20% by weight of the aniline compound by weight and 5 to 15% by weight of the dimethylcyanamide, clearly the amounts are chosen so that the solution remains free flowing and adapted to be refluxed. The heating may be at the reflux temperature of the solvent, e.g. 110°C in the case of toluene. The reaction may also be carried out by heating at a temperature below reflux and is then desirably accompanied by stirring, especially by vigorous stirring.

It may be desirable to have some water present in the reaction vessel, e.g. a small amount of water. Toluene has a very slight solubility in water.

The dehydroxylation reaction is carried out by heating the guanidinyl intermediate in the presence of alkali e.g. NaOH, for example by boiling it with alkali.

The scheme of the reaction according to this aspect of the invention is as follows:-

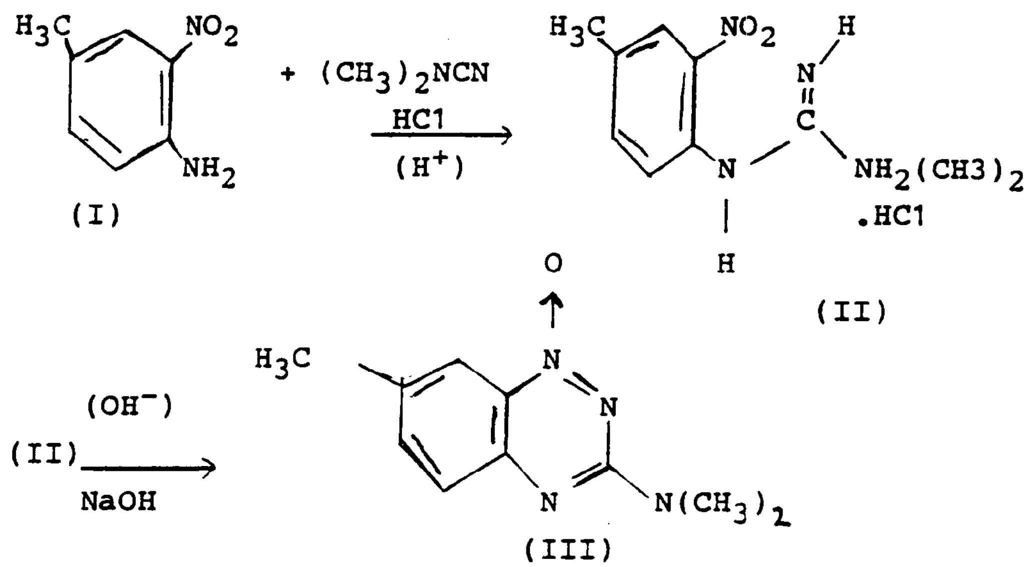


(I) (II) (III)

According to a preferred form of this aspect of present invention a process for the preparation of benzotriazine oxide comprises reacting dimethylcyanamide with 4-methyl-2-nitroaniline with the introduction of gaseous hydrogen chloride in excess non-polar solvent and at a temperature above 100°C and less than 160°C to obtain a precipitate of N,N-dimethyl-N'-(4-methyl-2-nitrophenyl) guanidine hydrochloride, which is then reacted with an alkali to produce the desired benzotriazine oxide derivative.

The reaction between the dimethyl cyanamide and the methylnitroaniline is preferably carried out at a temperature between 100°C and 145°C and more preferably at about 135°C. This reaction is preferably carried out under reflux conditions, in which case a solvent that forms a reaction mixture with an appropriate boiling point is chosen. Preferred examples are toluene, naphtha and p-xylene bpt 137-138°C, although in the latter two cases the temperature is preferably somewhat below the reflux temperature which is above the preferred temperature for this reaction.

The above reaction may be carried out under anhydrous conditions, or in the presence of a small quantity of water.

In order to allow the whole process to be performed in a single reaction vessel, the liquid residue may be decanted or pumped off from the precipitate of N,N-dimethyl-N'-(4-methyl-2-nitrophenyl) guanidine hydrochloride and an alkali such as NaOH added to the precipitate in the same reaction vessel, in order to bring about the ring closure dehydroxylation reaction.

The precipitated intermediate product N,N-dimethyl-N'-(4-methyl-2-nitrophenyl) guanidine hydrochloride may be purified before addition of the alkali which brings about the ring closure reaction. Such purification may involve washing and/or refluxing the precipitate with a suitable solvent, e.g. chloroform.

The molar ratio of the dimethylcyanamide to the 4-methyl-2-nitroaniline is preferably from about 0.8:1 to

about 2:1. Higher molar ratios within the above range seem to lead to higher yields of the desired product, but a molar ratio as high as 2.5:1 seems to be accompanied by a decrease in yield in comparison with a ratio of 2:1.

The reaction between dimethylcyanamide and 4-methyl-2-nitroaniline is preferably carried out with stirring.

The invention may be put into practice in various ways and a number of specific embodiments will be described to illustrate the invention with reference to the accompanying examples.

Examples 1 to 10 were laboratory scale preparations of N,N-dimethyl-N′-(4-methyl-2-nitrophenyl) guanidine hydrochloride (compound II above).

Example 1

4-methyl-2-nitroaniline (20 g, 0.13 mole) and dimethylcyanamide (10 g, 0.14 mole) were stirred in toluene (120 ml density 0.866 i.e. 104 g) and water (0.12 ml, 0.0067 mole), then heated to reflux to give a clear orange/red solution. The initial reaction solution at room temperature contained about 19% by weight of the aniline compound and 9.6% by weight of dimethyl cyanamide.

Hydrogen chloride gas (50 g total) was bubbled through the solution at reflux temperature (115°C) with stirring for 2 hours, to give a red liquor containing a yellow precipitate flecked with red oil. This mixture was refluxed for a further four hours and then allowed to cool.

The toluene was decanted off, the crude product slurried with chloroform (120 ml), and boiled under reflux for 5-10 minutes. The cooled mixture was filtered and the residue washed with ice cold chloroform (2 x 25 ml).

The yellow solid was dried at 50°C and weighed.IR, NMR and elemental analysis were perfomed.

Solvent was removed from the filtration liquors, by heating in the rotary evaporator, leaving a thick red/black oil.

| | |
|---|---|
| Typical yield of N,N-dimethyl N'-(4-methyl-2-nitrophenyl) guanidine hydrochloride | = 19.5 g (57%). |
| Melting point | = 222-224°C |
| Weight of residual oil | = 16 g. |

Examples 2 to 7

These were concerned with the effect of using various molar ratios of reactants.

Example 1 was repeated except that different molar ratios of dimethylcyanamide : 4-methyl-2-nitroaniline were used. (Example 1 used a ratio of 1.08:1).

The molar ratios used and the yields of N,N-dimethyl-N′-(4-methyl-2-nitrophenyl) guanidine hydrochloride obtained are shown in Table I below:

TABLE I

| Example | Molar ratio dimethylcyanamide : 4-methyl-2-nitroaniline | Yield |
|---|---|---|
| 2 | 1.5:1 | 23.5g (69%) |
| 3 | 2:1 | 25.2g (74%) |
| 4 | 2:1 | 26.7g (78%) |
| 5 | 2.5:1 | 21.0g (62%) |
| 6 | 1.75:1 | 23.5g (69%) |
| 7 | 1:1.2 or 0.83:1 | 22.0g (62%) |

Thus, a molar ratio of about 2:1 seems to give the highest yield.

Examples 8 to 10

These were concerned with the effect of using different solvents and reaction temperature.

The reaction described in Example 1 was repeated, subject to the variations shown in Table 2 below.

TABLE 2

| Example Yield | Temperature | Reaction solvent |
|---|---|---|
| 8 p-xylene | 130-135°C | 21.5 g (63%) |
| 9 p-xylene | 145°C | 20.6 g (60%) |
| 10 dry toluene (anhydrous reaction) | 115°C | 19.9 g (58%) |

Example 11

This was a laboratory-scale preparation of benzotriazine oxide (compound III).

Recrystallised N,N-dimethyl-N′-(4-methyl-2-nitrophenyl) guanidine hydrochloride (compound II above) (25 g, 0.097 mole) was boiled under reflux in molar sodium hydroxide solution (706 ml, 0.706 mole) for five minutes. The solution was allowed to cool to room temperature and the orange yellow solid filtered off, dried, ground to powder and washed with water (50 ml) and then dried at 70°C.

| | |
|---|---|
| Crude weight of benzotriazine oxide | = 18.8 g |
| Crude yield | = 95% |
| Melting point of crude compound | = 124-126°C. |

The product seemed pure from IR and NMR analysis and melting point, but 10 g of it was purified further by dissolving in boiling ethanol (15 ml), cooling, filtering and washing the solid with ice cold ethanol (10 ml) and drying at 70°C.

| | |
|---|---|
| Weight of purified benzotriazine oxide | = 9.6 g |
| Yield | = 91% |
| Melting point | = 126.5°C. |

Analysis of recrystallized product

| | Expected for $C_{10}H_{12}N_4O$ | Found in Sample |
|---|---|---|
| %C | 58.80 | 58.7 |
| %H | 5.92 | 5.9 |
| %N | 27.43 | 27.4 |

Example 12

This was a larger scale preparation of N,N-dimethyl-N'-(4-methyl-2-nitrophenyl) guanidine hydrochloride carried out on ten times the scale of Example 1.

Method: The 4-methyl-2-nitroaniline (400 g, 2.63 mole) and dimethylcyanamide (200 g, 2.85 mole) were dissolved in toluene (2400 ml) and heated to reflux (117°C) in a 5 litre flanged flask fitted with a stirrer, a temperature controller-recorder, a B34 Liebig condenser, an HCl gas inlet and an isomantle. Hydrogen chloride gas was bubbled through the refluxing mixture for a total of 12 hours over a period of 2 days. When it was necessary to interrupt the reaction at the end of the day, the reaction mixture was left to cool under a stream of nitrogen. At the completion of the run, the total quantity of hydrogen chloride used was 330 g (9.04 mole). The mixture was then refluxed for a further 6 hours.

During the course of the reaction, a white solid formed in the condenser and had to be removed occasionally to prevent blockage of the condenser.

After cooling the mixture, the solid was isolated by filtration and stirred with chloroform (1 litre) whilst boiling under reflux for 30 minutes. The mixture was cooled to 10°C, filteted and the solid on the filter was washed with chloroform (3 x 100 ml).

| | |
|---|---|
| Yield of N,N-dimethyl-N'-(4-methyl-2-nitrophenyl) guanidine hydrochloride | = 400.3 g (59%) |
| Melting point | = 224-225°C. |

Example 13

This was a larger scale preparation of benzotriazine oxide.

Benzotriazine oxide was prepared on sixteen times the scale of Example II using the product from Example

12 without further purification.

The product (440 g, 1.55 mole) and molar sodium hydroxide solution (11.24 litres) were mixed in a 20 litre flange-neck flask fitted with anchor stirrer, heating mantle, and reflux condenser. The orange/red suspension was boiled under reflux for 10 minutes then allowed to cool to 20°C with stirring. The solid was isolated by vacuum filtration and washed on the filter with water (2 x 400 ml). After drying at 70°C, the product was broken up and lightly ground in a mortar.

| | |
|---|---|
| Weight of benzotriazine oxide | = 295 g |
| Yield | = 93% |
| Melting point | = 125.5-126°C. |

Example 14

This is a comparison example.

4-methyl-2-nitroaniline (20 g, 0.13 mole) (compound I) and cyanamide ($NCNH_2$) (5.9 g, 0.14 mole) are stirred in toluene (120 ml density 0.866 i.e. 104 g) and water (0.12 ml, 0.0067 mole), then heated to reflux to give a clear orange/red solution. The cyanamide used is ordinary pure commercial material. The initial reaction solution at room temperature contains about 19% by weight of the aniline compound and 9.6% by weight of cyanamide.

Hydrogen chloride gas (50 g total) is bubbled through the solution at reflux temperature (115°C) with stirring for 2 hours, to give a red liquor containing a yellow precipitate flecked with red oil. This mixture is refluxed for a further four hours and then allowed to cool.

The toluene is decanted off, the crude product slurried with chloroform (120 ml), and boiled under reflux for 5-10 minutes. The cooled mixture is filtered and the residue washed with ice cold chloroform (2 x 25 ml).

The yellow solid is dried at 50°C.

Example 15

This is a comparison example.

The product of Example 14 recrystallized, namely amino-N′-(4-methyl-2-nitrophenyl) guanidine hydrochloride (compound II above) 25 g, 0.097 mole) is boiled under reflux in molar sodium hydroxide solution (706 ml, 0.706 mole) for five minutes. The solution is allowed to cool at room temperature and the orange yellow solid filtered off, dried, ground to powder and washed with water (50 ml) and then dried at 70°C.

Example 16

This is a comparison example.

4-methyl-2-nitroaniline (20 g, 0.13 mole) (compound I) and cyanamide ($NCNH_2$) (FLUKA) (5.9 g, 0.14 mole) are stirred in toluene (120 ml density 0.866 i.e. 104 g) and water (0.12 ml, 0.0067 mole), then heated to reflux at 115°C to give a clear red solution. The cyanamide used is ordinary pure material, supplied by FLUKA AG of Buchs, Switzerland under catalogue number 28330 and stated to have a purity of greater than 98% by weight, a m.pt. of 44-46°C, and to be stabilized with 0.05% by weight of phosphate and to contain less than 2% by weight water.

Hydrogen chloride gas is bubbled rapidly through the mixture with rapid stirring for 2 hours, to give a red liquor containing a yellowish precipitate. The mixture is refluxed for a further four hours and then allowed to cool.

The precipitate is filtered off and slurried with chloroform (120 ml), and boiled under reflux for 5-10 minutes, cooled to room temperature, filtered and washed with ice cold chloroform (2 x 50 mls).

The yellow solid is dried in vacuo in a dessicator to give 22gr of compound III (yield 75%). Recrystallization from ethanol gives a sample having a m.pt. of 205-208°C.

I.R. and N.M.R spectral analyses agree with the assigned structure. However, a satisfactory elemental analysis could not be obtained, probably due to the product containing some hydrochloride of the starting material.

Example 17

This is a comparison example.

Example 16 is repeated, but using a 100% molar excess of cyanamide. A yield of 75% of the crude product is obtained. Again a satisfactory elemental analysis could not be obtained.

Example 18

This is a comparison example.

The crude product of Example 17 was refluxed for 10 minutes with an excess of molar sodium hydroxide solution. The resulting product was 3-amino-7-methyl-1,2,4-benzotriazine-1-oxide (compound III) in 63% yield having a m.pt. of 271-273°C. I.R. and N.M.R. analyses indicated it to be pure. A sample recrystallized from a mixture of ethanol and one drop of acetic acid gave a satisfactory elemental analysis.

(Found: C 53.6%, H 4.4%, N 31.2%;)
(Expected: C 54.5%, H 4.5%, N 31.8%.)

Example 19

This is a comparison example.

The product of Examples 15 and 18 may then be converted to benzotriazine oxide by the following methylation procedure.

The 3-amino-7-methyl-1,2,4-benzotriazine-1-oxide (V) from Example 15 or Example 18 is converted to 3-dimethylamino-7-methyl-1,2,4-benzotriazine-1-oxide (III) as shown in the reaction sequence below. This is achieved by reaction of (V) with sodium nitrite and sulphuric acid at 30°C. The product (VI) undergoes tautomeric shift to a lactam (VII) and this is converted by reaction with phosphoryl chloride and dimethyl aniline at 120°C to compound (VIII). This is converted to compound (III) by refluxing with dimethylamine in alcohol.

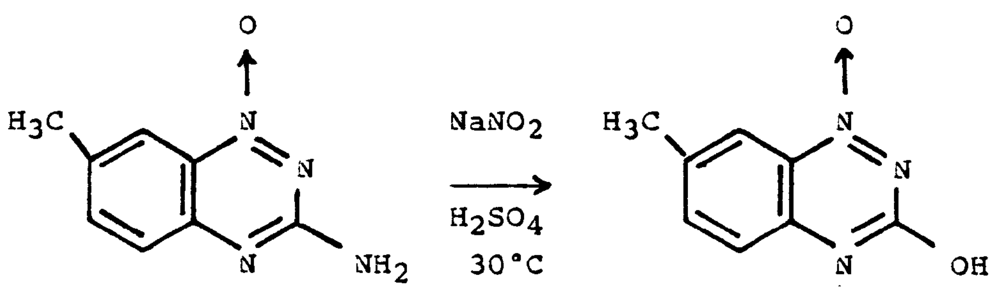


(V) (VI)

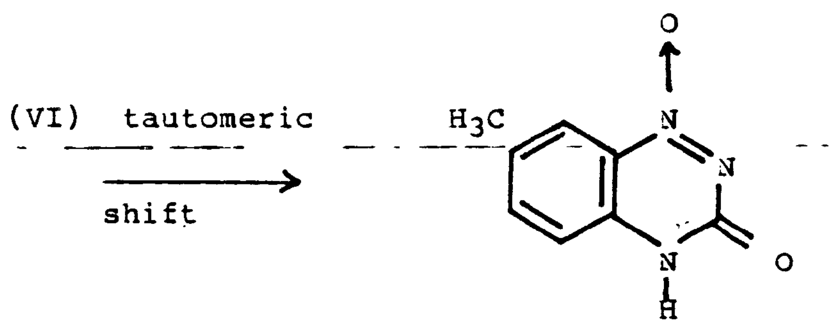


(VII)

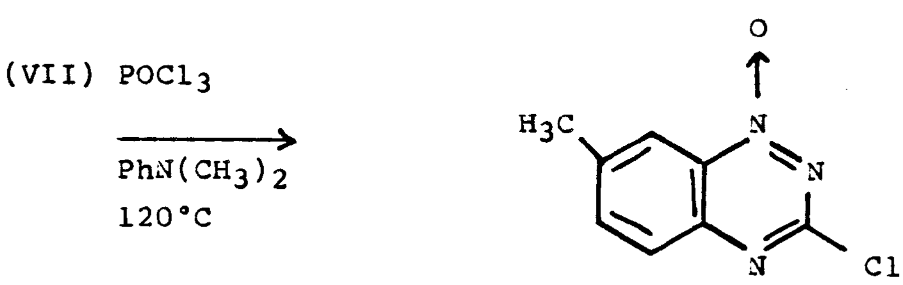


(VIII)

(VIII)

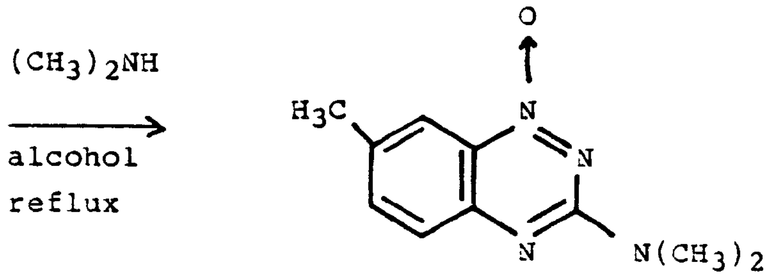


(III)

As mentioned above the present invention extends to azapropazone produced from benzotriazine oxide made by the processes of the present invention. Thus according to a further aspect of the present invention a process for making azapropazone comprises making benzotriazine oxide by a process in accordance with the present invention and then hydrogenating the benzotriazine oxide, e.g. with Pd/C catalyst, to produce a compound of the formula:

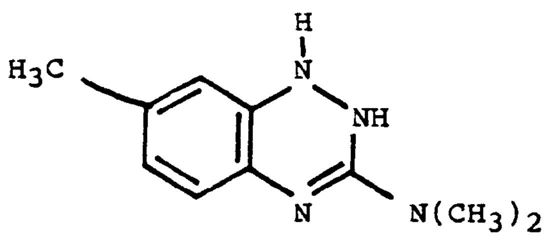

and then reacting this compound with mono-n-propyl malonic acid diethyl ester, namely:

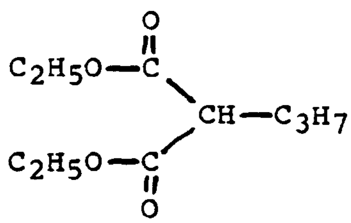

in the presence of sodium methoxide, $CH_3ONa$, and xylene to produce azapropazone, optionally as the dihydrate.

## Claims

1. A process for the preparation of 3-dimethylamino-7-methyl-1,2,4-benzotriazine-1-oxide which comprises providing a solution in a non-polar solvent which at 20°C contains 1 to 30% by weight of dimethylcyanamide and 5 to 50% by weight of 4-methyl-2-nitroaniline and heating the solution with the introduction of gaseous hydrogen chloride at a temperature above 100°C and less than 160°C to cause the cyanamide and the aniline compound to react to produce a precipitate of N,N-dimethyl-N′-(4-methyl-2-nitrophenyl) guanidine hydrochloride, which is then dehydroxylated by being heated with an alkali to produce 3-dimethylamino-7-methyl-1,2,4-benzotriazine-1-oxide.

2. A process for making azapropazone which comprises making 3-dimethylamino-7-methyl-1,2,4-benzotriazine-1-oxide by a process as claimed in claim 1 and then converting the 3-dimethylamino -7-methyl-1,2,4-benzotriazine-1-oxide to azapropazone in a manner known per se.

3. A process for making azapropazone which comprises making 3-dimethylamino-7-methyl -1,2,4-benzotriazine-1-oxide by a process as claimed in claim 1 and then hydrogenating the 3-dimethylamino-7-methyl-1,2,4-benzotriazine-1-oxide to produce a compound of the formula:

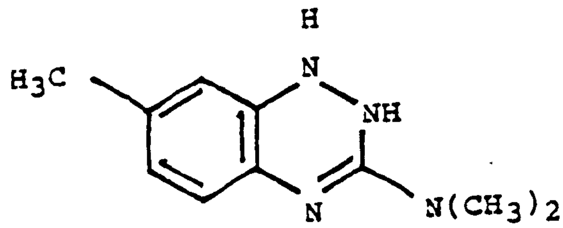

and then reacting this compound with mono-n-propyl malonic acid diethyl ester, namely:

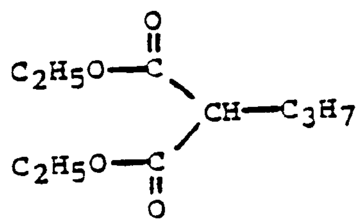

in the presence of sodium methoxide, $CH_3ONa$, and xylene to produce azapropazone, optionally as the dihydrate.

## Patentansprüche

1. Verfahren zur Herstellung von 3-Dimethylamino-7-methyl-1,2,4-benzotriazin-1-oxid, das das Vorsehen einer Lösung in einem nicht-polaren Lösungsmittel, die bei 20°C 1 bis 30 Gew.% Dimethylcyanamid und 5 bis 50 Gew.% 4-Methyl-2-nitroanilin enthält, und das Erhitzen der Lösung unter Einführung von gasförmigem Chlorwasserstoff bei einer Temperatur oberhalb 100°C und unterhalb 160°C, um zu bewirken, daß das Cyanamid und die Anilinverbindung unter Bildung eines Niederschlages von N,N-Dimethyl-N'-(4-methyl-2-nitrophenyl)-guanidinhydrochlorid reagieren, das dann durch Erhitzen mit einem Alkali unter Bildung von 3-Dimethylamino-7-methyl-1,2,4-benzotriazin-1-oxid dehydroxyliert wird, umfaßt.

2. Verfahren zum Herstellen von Azapropazon, das das Herstellen von 3-Dimethylamino-7-methyl-1,2,4-benzotriazin-1-oxid durch ein Verfahren nach Anspruch 1 und das anschließende Überführen des 3-Dimethylamino-7-methyl-1,2,4-benzotriazin-1-oxids in an sich bekannter Weise in Azapropazon umfaßt.

3. Verfahren zum Herstellen von Azapropazon, das das Herstellen von 3-Dimethylamino-7-methyl-1,2,4-benzotriazin-1-oxid durch ein Verfahren nach Anspruch 1 und dann das Hydrieren des 3-Dimethylamino-7-methyl-1,2,4-benzotriazin-1-oxids unter Bildung einer Verbindung der Formel

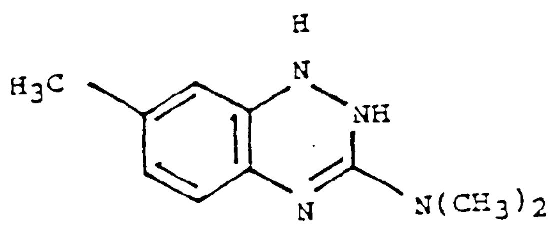

und das anschließende Umsetzen dieser Verbindung mit Mono-n-propylmalonsäurediäthylester, nämlich

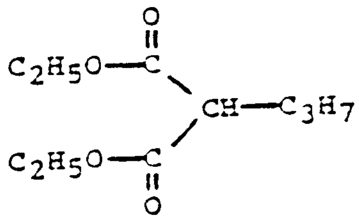

,

in Anwesenheit von Natriummethoxid, $CH_3ONa$ und Xylol unter Bildung von Azapropazon, gegebenenfalls als das Dihydrat, umfaßt.

## Revendications

1. Procédé de préparation du 3-diméthylamino-7-méthyl-1,2,4-benzotriazine-1-oxyde, qui comprend la formation d'une solution dans un solvant non polaire qui, à 20°C, contient 1 à 30% en poids de diméthylcyanamide et 5 à 50% en poids de 4-méthyl-2-nitroaniline et le chauffage de la solution tout en y introduisant du chlorure d'hydrogène gazeux à une température supérieure à 100°C et inférieure à 160°C pour amener le cyanamide et l'aniline à réagir en formant un précipité de chlorhydrate de N,N-diméthyl-N'-(4-méthyl-2-nitrophényl)guanidine qui est ensuite déshydroxylé par chauffage avec un alcali pour produire le 3-diméthylamino-7-méthyl-1,2,4-benzotriazine-1-oxyde.

2. Procédé de préparation de l'azopropazone, qui comprend la préparation du 3-diméthylamino-7-méthyl-1,2,4-benzotriazine-1-oxyde par un procédé suivant la revendication 1, puis la conversion du 3-diméthylamino-7-méthyl-1,2,4-benzotriazine-1-oxyde en azopropazone d'une manière connue en soi.

3. Procédé de préparation de l'azopropazone, qui comprend la préparation du 3-diméthylamino-7-méthyl-1,2,4-benzotriazine-1-oxyde par un procédé suivant la revendication 1, puis l'hydrogénation du 3-diméthylamino-7-méthyl-1,2,4-benzotriazine-1-oxyde pour produire un composé de formule :

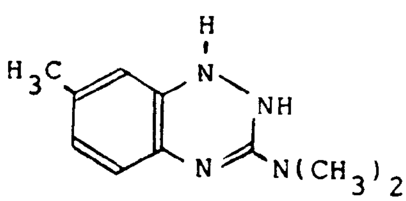

et ensuite la réaction de ce composé avec l'ester diéthylique de l'acide mono-n-propylmalonique, à savoir :

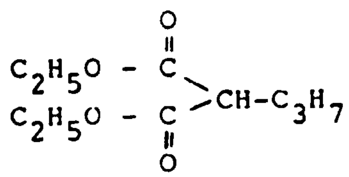

en présence de méthylate de sodium, $CH_3ONa$, et de xylène pour produire l'azapropazone, facultativement sous la forme du dihydrate.